# DEMANDE DE BREVET EUROPEEN

(11) **EP 0 815 832 A2**
(43) Date de publication de la demande: **07.01.1998**
(21) Numéro de dépôt: 97870096.1
(22) Date de dépôt: 01.07.1997
(51) Int. Cl.: A61K 7/16

(54) **Ensemble pour préparations pharmaceutiques destinées au traitement des dents et de la cavité buccale**

(30) Priorité: 01.07.1996 BE 9600605
(71) Demandeur: Cardon, Chris, 8370 Blankenberge (BE)
(72) Inventeur: Cardon, Chris, 8370 Blankenberge (BE)
(74) Mandataire: Van Malderen, Michel

(57) **Abrégé**

La présente invention concerne un ensemble ("kit" ou trousse) de préparations pharmaceutiques, caractérisé en ce que cet ensemble est constitué d'une série de constituants actifs destinés au traitement des dents sous une forme concentrée et une solution de base commune et compatible avec les constituants actifs précités, qui sert de support, d'excipient et de diluant inerte pouvant être utilisé pour l'ensemble de la série de constituants actifs.

## Description

### Objet de l'invention

La présente invention concerne un ensemble ("kit" ou trousse) pour des préparations pharmaceutiques destinées au traitement des dents et de la cavité buccale.

Lors de l'exercice de sa profession, le dentiste se trouve confronté à différentes affections ou atteintes telles que des gingivites, des saignements de la gencive, du tartre, des inflammations, de la sensibilité dentaire, etc.

Ceci entraîne la nécessité de devoir disposer sur place de différents moyens de traitement adaptés lors de la consultation dans son cabinet de dentisterie; de plus, il peut être amené à fournir au patient des préparations que celui-ci emporte pour poursuivre le traitement à domicile.

Le dentiste doit donc non seulement disposer d'une série de produits de traitement, mais il doit également être en mesure de réaliser pour le patient des préparations "sur mesure", c'est-à-dire généralement de concentration appropriée pour le patient.

Ceci doit de préférence pouvoir être réalisable de manière aussi simple que possible et les produits de base et les préparations que l'on prépare à partir de ceux-ci doivent naturellement être suffisamment stables; de plus, l'utilisateur doit pouvoir utiliser facilement lesdites préparations.

La forme d'application la plus courante pour un grand nombre de produits est celle du bain de bouche, c'est-à-dire une préparation aqueuse buccale avec laquelle le patient peut se rincer la cavité buccale, et en particulier ses dents et gencives.

Il est bien entendu avantageux, en particulier pour améliorer l'acceptabilité et la fidélité thérapeutiques, que des correcteurs de goût, c'est-à-dire des matières qui améliorent le goût du bain de bouche, soient inclus dans les préparations.

Un autre élément à prendre en considération est la coloration, généralement une couleur brunâtre, qui apparaît sur les dents lors de l'utilisation de divers bains de bouche.

On est amené à résoudre ce type de difficultés de manière efficace par diverses préparations, qui sont vendues prêtes à l'emploi. Ces compositions comportent généralement une quantité appropriée d'agents stabilisants, des correcteurs de goût et généralement des colorants permettant de compenser l'effet négatif de certains constituants actifs, etc.

Ces solutions prêtes à l'emploi, c'est-à-dire diluées à la concentration efficace, ou quelquefois destinées à être encore diluées avant chaque utilisation dans de l'eau par l'utilisateur, sont de toute manière relativement encombrantes lorsque le dentiste, au cours de sa pratique professionnelle, doit constituer un stock suffisant des différentes préparations appropriées, celui-ci se trouvant rapidement confronté à un manque de place.

Il n'est pas non plus concevable que le dentiste soit amené à préparer sur place des solutions des constituants actifs en les diluant par exemple l'aide d'eau et en y ajoutant les excipients nécessaires.

Le but visé par l'invention est donc de trouver une solution aux difficultés mentionnées, et en particulier de fournir un ensemble ("kit" ou trousse) de préparations pharmaceutiques caractérisé en ce que cet ensemble est constitué d'une série de constituants actifs destinés au traitement des dents sous une forme concentrée et une solution de base commune et compatible avec les constituants actifs précités, qui sert de support, d'excipient et de diluant inerte pouvant être utilisé pour l'ensemble de la série de constituants actifs.

En pratique, il suffit de disposer d'une série de préparations concentrées des constituants actifs les plus classiques pour les différents traitements que doit appliquer le dentiste et d'une solution de base commune, pour pouvoir préparer très rapidement un grand nombre de solutions du type bain de bouche.

A titre d'exemple, on peut considérer que six préparations différentes de constituants actifs sous forme concentrée sont contenus par exemple dans des flacons de 10 ml, tandis qu'un autre flacon contient une solution de base commune aux différents constituants actifs, ce flacon, d'une contenance totale par exemple de 250 ml au moins, contenant 240 ml de la solution de base.

En fonction des besoins, on peut donc verser la totalité de l'un des flacons avec le constituant actif approprié dans la solution de base en réalisant ainsi une dilution de 25 fois.

Bien entendu, la solution concentrée est soigneusement dosée, de manière à ce que la dilution habituellement souhaitée soit obtenue et que la solution finale, compte tenu des excipients présents, exerce l'effet thérapeutique souhaité.

Si le dentiste n'est pas satisfait de la dilution standard et qu'il veut, par exemple une dilution de l'ordre de 50 fois, il lui suffit de verser environ la moitié du flacon contenant le constituant actif qu'il a choisi dans le même flacon de 240 ml de solution de base.

Avantageusement, les flacons contenant les constituants actifs comportent des indications des niveaux permettant de mesurer leur contenu et donc de calculer les dilutions obtenues lorsque l'on n'ajoute qu'une partie des constituants actifs.

Cette façon de procéder entraîne de nombreux avantages, tant au niveau des gains de volume se traduisant par un encombrement moindre et des frais de transport plus réduits, de la facilité d'utilisation aussi bien pour le dentiste que pour le patient, et aussi, bien évidemment, une plus grande économie du fait que les préparations peuvent être réalisées au fur et à mesure de leur consommation.

Parmi les agents de traitement les plus actifs et les plus utilisés, on peut citer les dérivés (sels) de la chlorexidine, par exemple le diglyconate, le chlorhydrate ou l'acétate de chlorexidine.

La chlorexidine présente cependant l'inconvénient qu'elle provoque une coloration des dents, que la surface de la langue devient râpeuse et que l'on observe une perte des sensations gustatives.

Pour porter remède à ces difficultés, il est recommandé d'utiliser le phénoxyéthanol (phénoxétol). Le phénoxyéthanol renforce l'effet de la chlorexidine de manière que pour le même effet inhibant minimal, il suffit de n'utiliser qu'une concentration réduite en chlorexidine. Le phénoxyéthanol agit donc comme un excipient, et à ce titre, il est très avantageusement incorporé dans la solution de base.

Il convient de noter que des effets de dosage peuvent se produire. La précision de la dilution joue donc un rôle important, en particulier lorsque le phénoxyéthanol est contenu dans la solution de base et que la chlorexidine constitue un des constituants actifs.

Parmi les constituants actifs, on peut également mentionner le phosphate de calcium (pour la reminéralisation), le chlorure de zinc (pour des saignements de gencive) et les dérivés de fluor, de préférence sous concentration élevée dans des buts de reminéralisation.

Cette série de constituants actifs est déjà suffisante pour les atteintes les plus courantes dans la pratique.

Ainsi qu'il a été indiqué, selon l'invention, on prépare une série de ces constituants actifs sous forme concentrée, qui est livrée sous forme d'un ensemble ("kit" ou trousse) avec une solution de base unique qui est utilisée comme diluant ou support inerte pour la série de constituants actifs. Avantageusement, cet ensemble est contenu dans un emballage commun.

De préférence, la série de constituants actifs comporte un colorant, et dans ce cas, on prévoit avantageusement d'attribuer à chaque type de constituant actif une couleur qui lui est spécifique. La solution de base est, de préférence, non colorée et elle contient avantageusement des agents correcteurs de goût. L'avantage en est que le dentiste aussi bien que le patient, sur base de la couleur, peut vérifier si la dilution correcte est utilisée. On évite ainsi le danger d'utiliser une solution non diluée, du fait que la teinte et le goût attireraient l'attention du patient sur une possible erreur.

A titre d'exemple de composition pour la solution de base (c'est-à-dire la solution servant à la dilution de la préparation contenant les constituants actifs et qui est commune pour toutes ces constituants actifs), on peut citer :
- groupe du surfactant du type copolymère à bloc du polyéthylène et du polypropylène glycol SO-310 (0,1 à 0,0001%, et de préférence de l'ordre de 0,0005%);
- tween 20 (0,1 à 0,0001%, et de préférence de l'ordre de 0,0005%);
- phénoxétol BP (0,001 à 5%, et de préférence de l'ordre de 0,05%);
- xylitol USP (0,001 à 50%, et de préférence de l'ordre de 0,12%);
- correcteurs de goût q.s.; et
- eau épurée q.s.

Une telle solution est parfaitement stable et convient pour les différentes préparations de constituants actifs.

A titre d'exemples de solutions de constituants actifs, il est possible de préparer une série de préparations contenant les ingrédients cités. Elles sont répertoriées A - F ci-après :
A : digluconate de chlorexidine (cependant, le chlorhydrate et l'acétate conviennent également) en vue de préparer une solution qui est équivalente en MIC (Minimal Inhibiting Concentration) à une solution de 0,001 à 5%, de préférence 0,12%, de chlorexidine + colorants (patentblue)
B : digluconate de chlorexidine (cependant, le chlorhydrate et l'acétate conviennent également) en vue de préparer une solution qui est équivalente en MIC (Minimal Inhibiting Concentration) à une solution de 0,001 à 5%, de préférence 0,2%, de chlorexidine + colorants (patentblue)
C : digluconate de chlorexidine (cependant, le chlorhydrate et l'acétate conviennent également) en vue de préparer une solution qui est équivalente en MIC (Minimal Inhibiting Concentration) à une solution de 0,001 à 5%, de préférence 2%, de chlorexidine + colorants (patentblue)
D : solution de phosphate de calcium :
   - de 0,001 à 0,1 % calcium
   - de 0,001 à 0,1% phosphate
   - de 0,001 à 5% ions fluorures + colorants
E : chlorure de zinc de 0,001 à 10%, de préférence de l'ordre de 1%, + colorants
F : fluor de 0,001 à 5% + tampon bicarbonate de 0,001 à 5% + colorants

Toutes les concentrations indiquées, tant pour la solution de base que pour les solutions de constituants actifs, correspondent aux concentrations résultant d'une dilution de 25 fois de la préparation concentrée d'un constituant actif par la solution de base commune, comme c'est le cas par exemple en mélangeant 10 ml de la préparation concentrée à 240 ml de la solution de base. En d'autres mots, il s'agit donc de concentrations finales, la teneur en constituants actifs des solutions concentrées étant donc 25 fois supérieure à celle indiquée.

Ces différentes préparations de constituants actifs se présentent de préférence à l'état concentré sous un volume de 10 ml, et sont destinées en principe à une solution de base d'un volume de 240 ml si l'on veut réaliser une dilution de 25 fois.

On dispose ainsi d'un large éventail de possibilités de traitement, aussi bien pour ce qui concerne les différentes affections que l'on souhaite traiter que pour les différentes concentrations que le dentiste souhaite préparer.

On constate également que les solutions concentrées des constituants actifs, qui ne contiennent pratiquement pas d'excipients, sont parfaitement stables, aussi bien à l'état concentré que lorsqu'elles sont diluées à l'aide de la solution dite de base.

## Revendications

1. Ensemble ("kit" ou trousse) de préparations pharmaceutiques, caractérisé en ce que cet ensemble est constitué d'une série de constituants actifs destinés au traitement des dents sous une forme concentrée et une solution de base commune et compatible avec les constituants actifs précités, qui sert de support, d'excipient et de diluant inerte pouvant être utilisé pour l'ensemble de la série de constituants actifs.

2. Ensemble selon la revendication 1, caractérisé en ce que la série de constituants actifs comporte un colorant de couleur spécifique pour chaque type de constituant actif, et que la solution de base est non colorée et contient des agents correcteurs de goût, de manière que le dentiste ou le patient, sur base de la couleur, puisse vérifier si la dilution correcte est utilisée.

3. Ensemble selon la revendication 1 ou 2, caractérisé en ce que les constituants actifs sous forme concentrée sont contenus dans des flacons de 10 ml, tandis qu'un autre flacon contient une solution de base commune aux différents constituants actifs, ce flacon, d'une contenance totale par exemple de 250 ml au moins, contenant 240 ml de la solution de base.

4. Ensemble selon la revendication 3, caractérisé en ce que les flacons contenant les constituants actifs comportent des indications des niveaux permettant de mesurer leur contenu et donc de calculer les dilutions obtenues lorsque l'on n'ajoute qu'une partie des constituants actifs.

5. Ensemble selon l'une quelconque des revendications précédentes, caractérisé en ce que l'un des constituants actifs est un dérivé (sel) de la chlorexidine, en particulier le diglyconate, le chlorhydrate ou l'acétate de chlorexidine, et que du phénoxyéthanol agissant comme un excipient est incorporé dans la solution de base.

6. Ensemble selon l'une quelconque des revendications précédentes, caractérisé en ce que l'un des constituants actifs est au moins un constituant choisi parmi le groupe constitué par phosphate de calcium, le chlorure de zinc et les dérivés de fluor.

7. Ensemble selon l'une quelconque des revendications précédentes, caractérisé en ce qu'il est contenu dans un emballage commun.

8. Ensemble selon l'une quelconque des revendications précédentes, caractérisé en ce que la solution de base contient les ingrédients suivants :
- groupe du surfactant du type copolymère à bloc du polyéthylène et du polypropylène glycol SO-310 (0,1 à 0,0001%, et de préférence de l'ordre de 0,0005%);
- tween 20 (0,1 à 0,0001%, et de préférence de l'ordre de 0,0005%);
- phénoxétol BP (0,001 à 5%, et de préférence de l'ordre de 0,05%);
- xylitol USP (0,001 à 50%, et de préférence de l'ordre de 0,12%);
- correcteurs de goût q.s.; et
- eau épurée q.s.

9. Ensemble selon l'une quelconque des revendications précédentes, caractérisé en ce que les constituants actifs sont constitués par une série des constituants actifs suivants :
A :digluconate de chlorexidine (cependant, le chlorhydrate et l'acétate conviennent également) en vue de préparer une solution qui est équivalente en MIC (Minimal Inhibiting Concentration) à une solution de 0,001 à 5%, de préférence 0,12%, de chlorexidine + colorants (patentblue)
B :digluconate de chlorexidine (cependant, le chlorhydrate et l'acétate conviennent également) en vue de préparer une solution qui est équivalente en MIC (Minimal Inhibiting Concentration) à une solution de 0,001 à 5%, de préférence 0,2%, de chlorexidine + colorants (patentblue)
C :digluconate de chlorexidine (cependant, le chlorhydrate et l'acétate conviennent également) en vue de préparer une solution qui est équivalente en MIC (Minimal Inhibiting Concentration) à une solution de 0,001 à 5%, de préférence 2%, de chlorexidine + colorants (patentblue)
D :solution de phosphate de calcium :
- de 0,001 à 0,1 % calcium
- de 0,001 à 0,1% phosphate
- de 0,001 à 5% ions fluorures + colorants
E :chlorure de zinc de 0,001 à 10%, de préférence de l'ordre de 1%, + colorants
F :fluor de 0,001 à 5% + tampon bicarbonate de 0,001 à 5% + colorants;
toutes les concentrations indiquées, tant pour la solution de base que pour les solutions de constituants actifs, correspondent aux concentrations résultant d'une dilution de 25 fois de la préparation concentrée d'un constituant actif par la solution de base commune.

10. Utilisation de l'ensemble selon l'une quelconque des revendications précédentes, pour la préparation d'un bain de bouche.
